# EUROPÄISCHE PATENTSCHRIFT

(11) **EP 3 229 056 B1**
(45) Veröffentlichungstag und Bekanntmachung des Hinweises auf die Patenterteilung: **19.07.2023**
(21) Anmeldenummer: 17163768.9
(22) Anmeldetag: 30.03.2017
(51) Int. Cl.: G02B 27/00, A61B 1/055, G02B 23/24, A61B 1/00, A61B 1/04, A61B 1/06, A61B 1/07

(54) **OPTIKSYSTEM FÜR EIN ENDOSKOP**
LENS SYSTEM FOR AN ENDOSCOPE
SYSTÈME OPTIQUE POUR UN ENDOSCOPE

(30) Priorität: 08.04.2016 DE 102016106518
(43) Veröffentlichungstag der Anmeldung: 11.10.2017
(73) Patentinhaber: Henke-Sass, Wolf GmbH, 78532 Tuttlingen (DE)
(72) Erfinder: REHE, Oliver, 78573 Wurmlingen (DE)
(74) Vertreter: Patentanwälte Geyer, Fehners & Partner mbB

(56) Entgegenhaltungen:
- WO-A1-2015/135390
- JP-A- 2013 134 474

## Beschreibung

Die vorliegende Erfindung betrifft ein Optiksystem für ein Endoskop, wobei das Optiksystem die Merkmale des Oberbegriffs des Anspruches 1 aufweist.

Solche Optiksysteme sind in der Regel für Strahlung aus dem sichtbaren Wellenlängenbereich ausgelegt, um für diese Wellenlängen eine möglichst gute Abbildung realisieren zu können.

Es ist jedoch wünschenswert, auch Abbildungen mit Strahlung aus dem nahen Infrarotbereich durchführen zu können, da dies einen zusätzlichen Nutzen in der endoskopischen Diagnostik und bei endoskopischen Operationstechniken mit sich bringt. So können beispielsweise mittels der Fluoreszenzdiagnostik krankhafte Strukturen oder Durchblutungsstörungen besser erkannt und behandelt werden. Dazu wird in das zu untersuchende Gewebe oder Gefäßsystem ein Medikament (z.B. ICG = Indocyaningrün) eingebracht, das ein Fluoreszenzsignal mit maximaler Intensität bei einer Wellenlänge im Bereich von 830-840 nm aussendet, wenn es mit Strahlung einer vorbestimmten Wellenlänge (z.B. 805 nm) angeregt wird. Somit können beispielsweise zeitsequenziell Abbildungen bzw. Aufnahmen mit Licht aus dem sichtbaren Wellenlängenbereich und Abbildungen bzw. Aufnahmen mit Strahlung aus dem Infrarotbereich durchgeführt werden, die dann dem Benutzer entweder einzeln oder in Überlagerung auf einer Anzeigeeinheit dargestellt werden. So kann beispielsweise das Fluoreszenzsignal bzw. das entsprechende Bild in der entsprechenden Aufnahme mit Licht aus dem sichtbaren Wellenbereich markiert werden. Üblich ist beispielsweise ein grünes Einfärben der fluoreszierenden Bereiche.

Nachdem das Optiksystem in der Regel für Licht aus dem sichtbaren Wellenlängenbereich ausgelegt ist, liegt ein großer Farblängsfehler für Licht aus dem sichtbaren Wellenlängenbereich relativ zu Licht aus dem Infrarotbereich vor, was dazu führt, dass mit dem Licht aus dem nahen Infrarotbereich keine scharfe Abbildung durchführbar ist. Die Fokuslage für dieses Licht aus dem nahen Infrarotbereich ist so weit von der Fokuslage für Licht aus dem sichtbaren Wellenbereich beabstandet, dass ein Bildsensor, der in der Fokuslage für das Licht aus dem sichtbaren Wellenlängenbereich positioniert ist, keine scharfe Abbildung für Licht aus dem nahen Infrarotbereich durchführen kann.

Um nun dennoch die gewünschte scharfe Abbildung für Licht aus dem nahen Infrarotbereich durchführen zu können, kann man entweder manuell oder automatisch hin und her fokussieren. Ferner ist es bekannt, optische Baugruppen außerhalb des Endoskopes vorzusehen, die den Farblängsfehler korrigieren. Auch können Sensoren eingesetzt werden, die die Abbildungen mit Licht aus dem sichtbaren Wellenbereich und die Abbildung mit Licht aus nahen Infrarotbereich an verschiedenen Orten durchführen können. All diese Varianten sind sehr aufwendig.

Ferner ist es möglich, das Umkehrsystem des Optiksystems so auszulegen, dass der Farblängsfehler korrigiert wird. Dies führt jedoch zu einem sehr komplexen Umkehrsystem aus vielen verschiedenen optischen Linsen und Materialien, die zu großen Brechzahlsprüngen zwischen den Materialgrenzflächen führen. Solche Umkehrsysteme sind sehr aufwendig und auch sehr toleranzanfällig.

Die WO 2015/135390 A1 beschreibt ein Optiksystem mit den Merkmalen des Oberbegriffs des Anspruches 1.

Ausgehend hiervon ist es daher Aufgabe der vorliegenden Erfindung, ein Optiksystem für ein Endoskop bereitzustellen, mit dem die eingangs genannten Schwierigkeiten möglichst vollständig überwunden werden können.

Die Erfindung ist im Anspruch 1 definiert. Vorteilhafte Weiterbildungen sind in den abhängigen Ansprüchen angegeben.

Es wird somit erfindungsgemäß ausgenutzt, dass das Objektiv, das aufgrund seiner relativ kurzen Baulänge im Vergleich zum Umkehrsystem üblicherweise kaum zum Farblängsfehler beiträgt. Das Objektiv wird nun extra so ausgelegt, dass es einen sehr viel größeren Farblängsfehler als üblich aufweist, der darüber hinaus auch noch entgegengesetzt zum Farblängsfehler des Umkehrsystems ist. Damit wird eine Korrektur des durch das Umkehrsystem bedingten Farblängsfehlers erreicht. Des Weiteren sind die optischen Anforderungen an das Umkehrsystem geringer als in einem Fall, bei dem die Farblängsfehlerkorrektur nur durch das Umkehrsystem durchgeführt wird, so dass das Umkehrsystem des erfindungsgemäßen Optiksystems beispielsweise mit weniger optischen Elementen gebildet werden kann.

Der erste Farblängsfehler kann sich auch auf mehrere vorbestimmte Wellenlängen (oder ein oder mehrere Wellenlängenbereiche) aus dem sichtbaren Spektrum (bzw. sichtbaren Wellenlängenbereich) und die vorbestimmte Wellenlänge aus dem nahen Infrarotbereich oder mehrere vorbestimmte Wellenlängen (oder ein oder mehrere Wellenlängenbereiche) aus dem nahen Infrarotbereich beziehen. Ferner können sich der erste und zweite Farblängsfehler auf mehrere Wellenlängen (oder ein oder mehrere Wellenlängenbereiche) aus dem nahen Infrarotbereich und die vorbestimmte Wellenlänge aus dem sichtbaren Spektrum oder mehrere Wellenlängen (oder ein oder mehrere Wellenlängenbereiche) aus dem sichtbaren Spektrum beziehen. Man kann auch sagen, dass der erste und zweite Farblängsfehler jeweils einen Verlauf aufweist, wobei die Verläufe so entgegengesetzt sind, dass sie in der Summe kleiner werden.

Bei dem erfindungsgemäßen Optiksystem können alle gekrümmten Materialgrenzflächen des Objektivs sphärisch gekrümmt sein. Dies vereinfacht die Herstellung des Objektivs.

Das Umkehrsystem, das auch als Stablinsensystem bezeichnet werden kann, kann mehrere hintereinander angeordnete Umkehrstufen aufweisen. Bevorzugt führt jede Umkehrstufe eine Zwischenabbildung durch. Die Umkehrstufen sind bevorzugt so hintereinander angeordnet, dass durch aufeinander folgende Zwischenabbildungen eine Weiterleitung des Zwischenbilds in der distalen Zwischenbildebene zur proximalen Zwischenbildebene realisiert wird.

Mindestens eine der Umkehrstufen kann zwei direkt miteinander verbundene Linsen aufweisen, deren Materialien so gewählt sind, dass sich die Brechzahlen der Materialien um nicht mehr als 0,3 unterscheiden. Insbesondere kann mindestens eine der Umkehrstufen so ausgebildet sein, dass jeder Brechzahlsprung aller direkt miteinander verbundenen Linsen dieser Umkehrstufe kleiner oder gleich 0,3 ist. Die Verbindung der Linsen kann durch z.B. Verkitten, Verkleben oder Ansprengen realisiert sein.

Durch diese geringen Brechzahlsprünge werden die Fresnel-Verluste reduziert, so dass die Transmission erhöht werden kann. Dies ist insbesondere für die Signale aus dem nahen Infrarotbereich von Vorteil, da diese in der Regel relativ schwach sind.

Als Brechzahlen bzw. als Brechungsindizes werden hier in der Regel n_{d} verwendet und somit die Brechzahlen für die Wellenlänge von 587,56 nm.

Das sichtbare Spektrum bzw. der sichtbare Wellenlängenbereich ist hier insbesondere der Wellenlängenbereich von 400-700 nm. Als naher Infrarotbereich wird hier insbesondere der Bereich von 710-3000 nm, 710-900 nm oder auch 780-900 nm verstanden.

Bei dem erfindungsgemäßen Optiksystem kann mindestens eine Umkehrstufe eine Linse mit einer asphärischen Grenzfläche aufweisen. Damit ist eine gute Korrektur der sphärischen Aberration möglich.

Die vorbestimmte Wellenlänge aus dem sichtbaren Spektrum kann insbesondere eine Wellenlänge aus dem Bereich von 400-700 nm und die vorbestimmte Wellenlänge aus dem nahen Infrarotbereich kann eine Wellenlänge aus dem Bereich von 710-900 nm sein.

Die mehreren Umkehrstufen des Umkehrsystems können insbesondere so hintereinander angeordnet sein, dass die Zwischenbildebene einer Umkehrstufe mit der Zwischenbildebene zusammenfällt, aus der die nachfolgende Umkehrstufe ein Zwischenbild in die nächste Zwischenbildebene dieser nachfolgenden Umkehrstufe abbildet.

Das Umkehrsystem kann als symmetrisches oder als unsymmetrisches Stablinsensystem ausgebildet sein. Insbesondere kann das Umkehrsystem zwei oder mehr Umkehrstufen aufweisen, wobei genau eine Umkehrstufe oder mindestens eine der Umkehrstufen als unsymmetrische Umkehrstufe ausgebildet ist. Insbesondere können zwei Umkehrstufen oder alle Umkehrstufen als unsymmetrische Umkehrstufen ausgebildet sein.

Ferner kann eine erste Umkehrstufe, die am nähesten an der distalen Zwischenbildebene liegt, einen Abbildungsmaßstab von größer als 1 aufweisen. Eine zweite Umkehrstufe, die am nähesten zur ersten Umkehrstufe liegt, kann einen Abbildungsmaßstab von kleiner als 1 aufweisen. Insbesondere können die erste und zweite Umkehrstufe zusammen einen Abbildungsmaßstab von 1 aufweisen.

Ferner kann eine der Umkehrstufen eine einer der Zwischenbildebene zugewandte gekrümmte Grenzfläche aufweisen, die asphärisch gekrümmt ist. Die asphärische Krümmung kann dabei eine Rotationssymmetrie aufweisen. Es ist jedoch auch möglich, dass die asphärische Krümmung keine Rotationsymmetrie aufweist und in den zwei Hauptabschnitten unterschiedlich gekrümmt ist.

Bevorzugt weist mindestens eine Stablinse jedes Umkehrsystems mindestens eine gekrümmte (z.B. sphärisch oder asphärisch gekrümmte) Materialgrenzfläche auf.

Jede Umkehrstufe kann insbesondere zumindest zwei Stablinsen aufweisen, wobei die Stablinsen einer Umkehrstufe gleich oder verschieden ausgebildet werden können. Wenn die Stablinsen gleich ausgebildet sind, können sie relativ zueinander gleichsinnig oder gegensinnig angeordnet sein. Zumindest eine der Stablinsen kann aus zumindest zwei, drei oder vier Teilen aufgebaut sein. Es ist natürlich auch möglich, dass sie aus mehr als vier Teilen aufgebaut ist. Insbesondere kann sie als Kittglied ausgebildet sein. Ferner ist es möglich, dass zumindest eine der Stablinsen einstückig ausgebildet ist.

Zumindest zwei Umkehrstufen des Umkehrsystems können zueinander symmetrisch angeordnet sein.

Das erfindungsgemäße Optiksystem ist insbesondere für starre Endoskope oder Endoskope mit starrem Endoskopschaft vorgesehen. Das erfindungsgemäße Optiksystem kann für einen Geradeausblick-Endoskop oder für ein Endoskop mit geneigter Blickrichtung ausgebildet sein. Ferner ist es möglich, dass das Optiksystem so ausgebildet ist, dass die Blickrichtung änderbar ist.

Die Stablinsen der Umkehrstufen können einen Durchmesser im Bereich von 1 bis 7,5 mm, 1 bis 6,5 mm und insbesondere im Bereich von 1,7 bis 5 mm aufweisen. Die Länge einer Umkehrstufe kann im Bereich von 30 bis 120 mm oder im Bereich von 40 bis 80 mm liegen.

Die Anzahl der Umkehrstufen kann im Bereich von 1 bis 11 oder 2 bis 11 Umkehrstufen liegen. Bevorzugt ist eine ungerade Anzahl von Umkehrstufen. Somit sind insbesondere eine Umkehrstufen, drei, fünf, sieben, neun und eif Umkehrstufen möglich. Natürlich ist es auch möglich, eine gerade Anzahl von Umkehrstufen vorzusehen.

Das Umkehrsystem kann insbesondere einen Abbildungsmaßstab im Bereich von 0,5 bis 2 aufweisen. Größere oder kleinere Werte sind ebenfalls möglich.

Als Material für das Umkehrsystem und/oder das Objektiv können Glas- und Kunststoffmaterialien verwendet werden.

Erfindungsgemäß beträgt die vorbestimmte Wellenlänge aus dem sichtbaren Spektrum 540 nm und die vorbestimmte Wellenlänge aus dem nahen Infrarotbereich 840 nm, wobei der erste Farblängsfehler im Bereich von 35 bis 65 µm (oder auch im Bereich von 40 bis 60 µm) und der zweite Farblängsfehler im Bereich von -4 bis -0,5 µm (oder auch im Bereich von -3,5 bis -1 µm) liegt.

Ferner kann das Verhältnis des ersten Farblängsfehlers zum zweiten Farblängsfehler (für grün zu infrarot) im Bereich von -55 bis -5 liegen.

Das Umkehrsystem kann einen dritten Farblängsfehler für die Wellenlänge von 540 nm zur Wellenlänge von 460 nm und einen vierten Farblängsfehler für die Wellenlänge von 540 nm zur Wellenlänge von 640 nm aufweisen und das Objektiv kann einen fünften Farblängsfehler für die Wellenlänge von 540 nm zur Wellenlänge von 460 nm und einen sechsten Farblängsfehler für die Wellenlänge von 540 nm zur Wellenlänge von 640 nm aufweist. Das Verhältnis des dritten zum vierten Farblängsfehler kann im Bereich von -1,1 bis -0,8 und das Verhältnis des fünften zum sechsten Farblängsfehler kann im Bereich von -4,5 bis -3 liegen.

Das Umkehrsystem und das Objektiv sind bevorzugt so ausgelegt, dass der dritte Farblängsfehler einen negativen Wert und der fünfte Farblängsfehler einen positiven Wert aufweist.

Das Objektiv kann einen siebten Farblängsfehler für die Wellenlänge von 460 nm zur Wellenlänge von 840 nm und einen Bildwinkel alpha im Objektraum aufweisen. Das Produkt von sin(alpha/2) mit dem siebten Farblängsfehler kann im Bereich von 18 bis 26 µm liegen.

Bei dem erfindungsgemäßen Optiksystem kann die vorbestimmte Wellenlänge aus dem sichtbaren Spektrum 540 nm und die vorbestimmte Wellenlänge aus dem nahen Infrarotbereich 840 nm betragen und das Umkehrsystem kann n optische Elemente aufweisen. Das Produkt aus der Anzahl n der optischen Elemente mit dem ersten Farblängsfehler kann im Bereich von 1500 bis 2000 µm liegen.

Ferner kann das Objektiv dem distalen Zwischenbild einen solchen Farblängsfehler einprägen, dass die axiale Lage bzw. Fokuslage für eine Wellenlänge von 840 nm zwischen den axialen Lagen bzw. Fokuslage für 540 nm und 640 nm liegt.

Bei dem Objektiv kann der Verlauf der Fokuslage für die Wellenlängen 640 nm, 540 nm und 460 nm negativ sein. Beim Umkehrsystem kann der Verlauf der Fokuslage im Wellenlängenbereich von 640 nm bis 460 nm positiv sein (hier bevorzugt annähernd linear). Ferner kann der Verlauf der Fokuslage für die Wellenlängen 460 nm, 540 nm, 640 nm zum nahen Infrarotbereich positiv sein. Man kann auch sagen, dass der Verlauf der Farborte blau, grün, rot zum nahen Infrarotbereich positiv ist.

Beim Objektiv kann der Extremwert der Fokuslage in Abhängigkeit der Wellenlänge im Bereich von 640 bis 680 nm liegen.

Ferner wird ein Endoskop mit einem erfindungsgemäßen Optiksystem (einschließlich der Weiterbildungen des erfindungsgemäßen Optiksystems) bereitgestellt.

Das Endoskop kann ferner eine dem Umkehrsystem nachgeordnete Optikeinheit, wie z.B. ein Okular, aufweisen. Des Weiteren kann das Endoskop einen Kameraanschluss aufweisen, an dem lösbar eine Kamera direkt oder über einen Koppler befestigt werden kann. Des Weiteren kann das Endoskop einen Beleuchtungslichtanschluss aufweisen, über den Beleuchtungslicht zugeführt werden kann, das dann im Endoskop bis zum distalen Ende des Schaftes des Endoskops geleitet wird, um ein vor dem distalen Ende befindliches Objekt zu beleuchten.

Insbesondere kann das Endoskop als Endoskop mit einem starren Schaft ausgebildet sein, in dem das erfindungsgemäße Optiksystem angeordnet ist.

Die Aufnahmen mit Licht aus dem sichtbaren Wellenlängenbereich und Licht aus dem nahen Infrarot werden mit dem Endoskop bevorzugt zeitsequenziell durchgeführt.

Das erfindungsgemäße Endoskop kann weitere dem Fachmann bekannte Merkmale aufweisen, die zum Betrieb des Endoskops notwendig sind.

Es versteht sich, dass die vorstehend genannten und die nachstehend noch zu erläuternden Merkmale nicht nur in den angegebenen Kombinationen, sondern auch in anderen Kombinationen oder in Alleinstellung einsetzbar sind, ohne den Rahmen der vorliegenden Erfindung zu verlassen.

Nachfolgend wird die Erfindung beispielsweise anhand der beigefügten Zeichnungen, die auch erfindungswesentliche Merkmale offenbaren, noch näher erläutert. Es zeigen:
- Fig. 1: eine schematische Darstellung einer ersten Ausführungsform des erfindungsgemäßen Optiksystems in einem Endoskop;
- Fig. 2: eine schematische Darstellung einer zweiten Ausführungsform des erfindungsgemäßen Optiksystems in einem Endoskop;
- Fig. 3: eine Darstellung der Abhängigkeit der Fokuslage von der Wellenlänge für ein herkömmliches Optiksystem;
- Fig. 4: eine schematische Darstellung des Aufbaus des Umkehrsystems des erfindungsgemäßen Optiksystems;
- Fig. 5: eine schematische Darstellung der durch das Umkehrsystem gemäß Fig. 4 bedingten Fokuslage in Abhängigkeit der Wellenlänge;
- Fig. 6: eine schematische Darstellung des optischen Aufbaus des Objektivs 4 des erfindungsgemäßen Optiksystems;
- Fig. 7: eine Darstellung der durch das Objektiv bedingten Fokuslage in Abhängigkeit der Wellenlänge;
- Fig. 8: eine schematische Darstellung der durch das erfindungsgemäße Optiksystem bedingten Fokuslage in Abhängigkeit der Wellenlänge;
- Fig. 9: eine schematische Darstellung der durch ein Umkehrsystem eines bekannten Endoskopes bedingten Fokuslage in Abhängigkeit der Wellenlänge;
- Fig. 10: eine schematische Darstellung der durch ein Objektiv eines bekannten Optiksystems bedingten Fokuslage in Abhängigkeit der Wellenlänge;
- Fig. 11: eine schematische Darstellung der Fokuslage eines bekannten Optiksystems in Abhängigkeit der Wellenlänge.

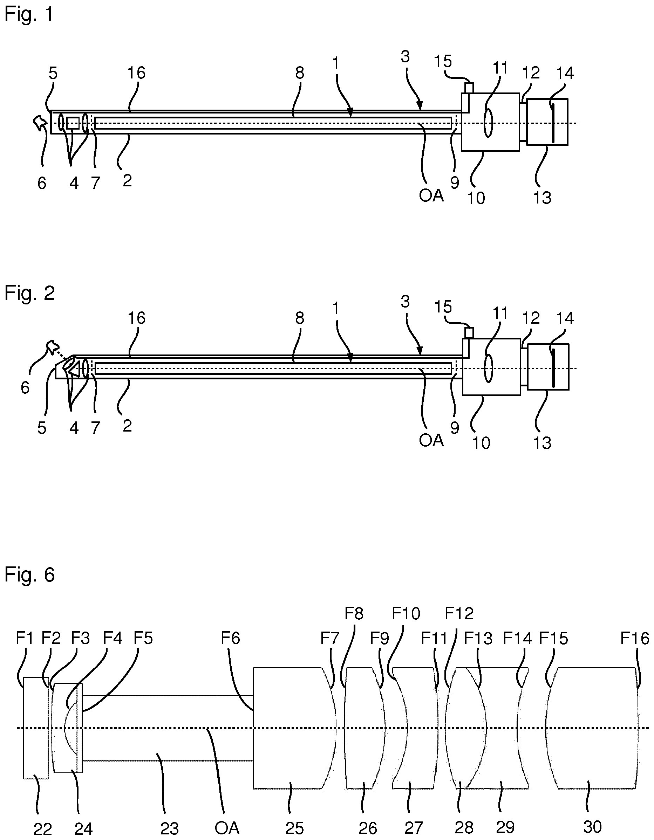

Bei der in Fig. 1 gezeigten Ausführungsform ist das erfindungsgemäße Optiksystem 1 in einem starren Schaft 2 eines schematisch dargestellten Endoskops 3 angeordnet.

Das Optiksystem 1 umfasst ein schematisch dargestelltes Objektiv 4, das ein vor einem distalen Ende 5 befindliches Objekt 6 als distales Zwischenbild in eine distale Zwischenbildebene 7 abbildet, und ein schematisch dargestelltes Umkehrsystem 8, das das distale Zwischenbild als proximales Zwischenbild in eine proximale Zwischenbildebene 9 abbildet. Das Objektiv 4 und das Umkehrsystem 8 weisen eine gemeinsame optische Achse OA auf.

Das Endoskop 3 umfasst ferner ein Hauptteil 10, in dem eine weiter Optik angeordnet sein kann, wie z. B. das schematisch dargestellte Okular 11, sowie einen Kameraanschluss 12. An dem Kameraanschluss 12 kann eine Kamera 13 lösbar mit dem Hauptteil 10 verbunden werden, wie schematisch in Fig. 1 dargestellt ist. Die Kamera 13 kann eine (nicht gezeigte) Optik aufweisen. Ferner enthält die Kamera 13 einen flächigen Bildsensor 14, der beispielsweise als CCD-Sensor oder als CMOS-Sensor ausgebildet ist. Die Kamera kann nicht nur, wie in Fig. 1 gezeigt ist, direkt mit dem Kameraanschluss 12 verbunden sein. Es ist auch möglich, dass zwischen dem Kameraanschluss 12 und der Kamera 13 ein (nicht gezeigter) Koppler zwischengeschaltet ist, der seinerseits eine Optik enthalten kann.

Das in Fig. 1 gezeigte Endoskop 3 ist als Geradeausblick-Endoskop ausgebildet, da mit ihm ein Objekt 6 aufgenommen werden kann, das in der Richtung, in der sich der Endoskopschaft 2 erstreckt, vor dem distalen Ende 5 positioniert ist. Es ist jedoch auch möglich, dass das Endoskop 3 quasi in eine andere Richtung als die Richtung blickt, in der sich der Endoskopschaft 2 erstreckt. Dies ist schematisch in Fig. 2 gezeigt, wobei hier die Blickrichtung um 45° gegenüber der Erstreckungsrichtung des Endoskopschafts 2 nach oben geneigt ist. Bei den Endoskopen gemäß Fig. 1 und 2 ist das Umkehrsystem 8 jeweils gleich ausgebildet. Lediglich das Objektiv 4 unterscheidet sich darin, dass im Objektiv die gewünschte Umlenkung, sofern notwendig, stattfindet, wie nachfolgend noch im Detail beschrieben wird.

Ferner kann das Endoskop z. B. am Hauptteil 10 einen Beleuchtungsanschluss 15 aufweisen, über den die gewünschte Beleuchtungsstrahlung dem Endoskop 3 zugeführt werden kann. Der Beleuchtungsanschluss 15 ist mit einem schematisch eingezeichneten Lichtleiter 16 verbunden, der sich bis zum distalen Ende 5 des Endoskopes 3 erstreckt und die geführte Strahlung zur Beleuchtung des Objektes 6 abgibt.

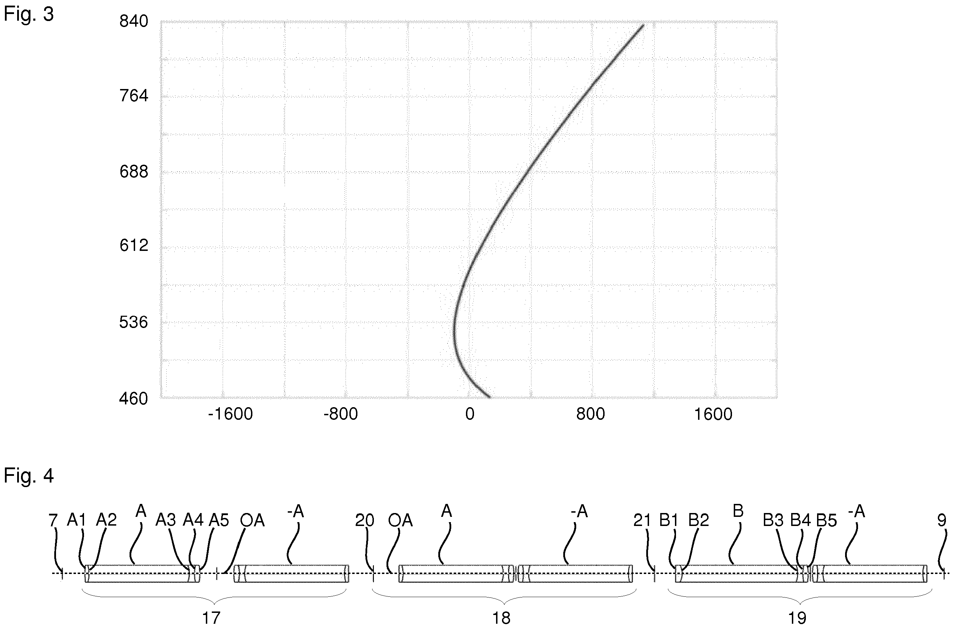

Im Betrieb des Endoskopes 3 wird das abzubildende Objekt 6 mit der Strahlung vom Lichtleiter 16 beleuchtet und das beleuchtete Objekt 6 wird über das Objektivsystem 1 in die proximale Zwischenbildebene 9 abgebildet und dort mittels des Okulars 11 in die Ebene des Bildsensors 14, so dass der Bildsensor 14 ein scharfes Bild des Objektes 6 aufnehmen kann. Bekannte Endoskope sind üblicherweise für den Wellenlängenbereich von ca. 400 bis 700 nm ausgelegt, der für das menschliche Auge sichtbar ist. Dabei soll der wellenlängenabhängige Farblängsfehler für den sichtbaren Wellenbereich möglichst gering sein. In Fig. 3 ist die erzeugte Fokuslage bei einem herkömmlichen Endoskop entlang der x-Achse in µm in Abhängigkeit der Wellenlänge entlang der y-Achse in nm aufgetragen. Dabei bedeutet eine Fokuslage von 0 µm, dass der Ort der schärfsten Abbildung mit der Ebene zusammenfällt, in der der Bildsensor 14 liegt. Eine Fokuslage von größer als 0 µm bedeutet, dass der Ort der schärfsten Abbildung hinter (und somit weiter rechts in Fig. 1 und 2) der Ebene ist, in der der Bildsensor 14 liegt. Entsprechend ist eine negative Fokuslage eine Abweichung von der optimalen Fokuslage in Richtung zum distalen Ende 5 hin. Wie dieser Darstellung entnommen werden kann, liegen in Bereich von 460 bis 700 nm Abweichungen im Bereich von ca. -100 µm bis 400 µm vor. Dies ist für eine ausreichend gute Abbildung mittels des Sensors 14 noch akzeptabel.

In Fig. 3 sind die Fokuslagen jedoch auch für Wellenlängen von größer als 700 nm und insbesondere für den Bereich von 700 bis 840 nm gezeigt. Dieser Bereich ab 700 nm und beispielsweise von 700 bis 900 nm oder auch von 780 bis 3000 nm wird als naher Infrarotbereich bezeichnet und wird für die Endoskopie zunehmend interessant, da z. B. zusätzlich zu einer Abbildung mit Licht aus dem sichtbaren Wellenlängenbereich eine Fluoreszenzdiagnostik durchgeführt werden kann. Es kann beispielsweise in zu untersuchendes Gewebe oder ein zu untersuchendes Gefäßsystem ein Fluoreszenzstoff, wie z.B. ICG (Indocyaningrün) eingespritzt werden, der bei Anregung mit Licht einer Wellenlänge von ca. 805 nm ein Fluoreszenzsignal mit größerer Wellenlänge abstrahlt, dessen Wellenlängen z.B. 830 bis 840 nm betragen. Es ist daher gewünscht, auch ein Bild mit Strahlung aus dem nahen Infrarotbereich mittels der Kamera 13 aufnehmen zu können. Wie der Darstellung in Fig. 3 jedoch entnommen werden kann, beträgt der Farblängsfehler bezogen z. B. auf die Wellenlänge von 540 nm und die Wellenlänge von 840 nm (und somit die Differenz der Fokuslagen für diese beiden Wellenlängen) etwa 1200 µm. Bei den Wellenlängen 460 nm und 840 nm beträgt der Farblängsfehler etwa 1000 µm. Dies führt dazu, dass keine scharfe Abbildung mit den Fluoreszenzsignalen durchgeführt werden kann.

Der beschriebene Farblängsfehler wird hauptsächlich durch das Umkehrsystem erzeugt, da wegen der notwenigen Länge des Schaftes 2 viele Stablinsen mit unterschiedlichen Materialien notwendig sind, die alle zum Farblängsfehler beitragen. Erfindungsgemäß ist daher das Optiksystem 1 so ausgelegt, dass der durch das Umkehrsystem 8 bedingte Farblängsfehler durch einen entgegengesetzten Farblängsfehler des Objektivs 4 möglichst stark verringert wird. Das Objektiv 4 trägt in der Regel aufgrund der sehr viel kürzeren Ausdehnung im Vergleich zum Umkehrsystem 8 kaum zum Farblängsfehler bei. Erfindungsgemäß wird das Objektiv 4 nun so ausgelegt, dass es einen sehr viel größeren Farblängsfehler als üblich aufweist, der jedoch entgegensetzt zum Farblängsfehler des Umkehrsystems 8 ist, so dass der resultierende Farblängsfehler im Zwischenbild in der proximalen Zwischenbildebene 9 verringert ist zu dem Fall, bei dem das Objektiv keinerlei Farblängsfehler verursachen würde und der Farblängsfehler nur durch das Umkehrsystem bedingt wäre.

Bei dem erfindungsgemäßen Optiksystem 1 ist das Umkehrsystem 8, wie in Fig. 4 gezeigt ist, aus drei hintereinander angeordneten Umkehrstufen 17, 18, 19 gebildet, die jeweils ein Zwischenbild in eine nächste Zwischenbildebene abbilden. So bildet die erste Umkehrstufe 17 das in der distalen Zwischenbildebene 7 liegende Zwischenbild in eine zweite Zwischenbildebene 20 ab. Die zweite Umkehrstufe 18 bildet das in der zweiten Zwischenbildebene 20 liegende Zwischenbild in eine dritte Zwischenbildebene 21 ab. Die dritte Umkehrstufe 19 bildet das Zwischenbild aus der dritten Zwischenbildebene 21 in die proximale Zwischenbildebene 9 ab.

Die drei Umkehrstufen 17-19 sind somit so hintereinander angeordnet, dass ein in der distalen Zwischenbildebene 7 liegendes Zwischenbild (über die jeweils nächste Zwischenbildebene 20 und 21) in die proximale Zwischenbildebene 9 abgebildet wird. Nachdem jede Umkehrstufe 17-19 bei der Abbildung des Zwischenbildes ein umgekehrtes Zwischenbild erzeugt und eine ungerade Anzahl von Umkehrstufen 17-19 vorgesehen sind, wird das in der distalen Zwischenbildebene 7 liegende Zwischenbild des Objektes 6 als umgekehrtes Zwischenbild in die proximale Zwischenbildebene 9 abgebildet.

Die zwei Umkehrstufen 17 und 18 weisen jeweils zwei Stablinsen vom Typ A auf. Die Umkehrstufe 19 weist eine Stablinse vom Typ A und eine Stablinse vom Typ B auf. Alle Stablinsen vom Typ A haben sphärische Radien und sind identisch aufgebaut. Die mit -A in Fig. 4 bezeichnete Stablinse ist identisch mit der Stablinse vom Typ A, lediglich anders herum eingebaut. Die Stablinsen vom Typ A weisen die Grenzflächen A1 (sphärisch positiv gekrümmt), A2 (sphärisch negativ gekrümmt), A3 (sphärisch negativ gekrümmt), A4 (sphärisch positiv gekrümmt) und A5 (sphärisch negativ gekrümmt) auf. Das Material zwischen den Flächen A1 und A2 weist eine Brechzahl n_{d} = 1,8 und eine Abbe-Zahl v_{d} = 29,9 auf. Das Material zwischen den Grenzflächen A2 und A3 weist eine Brechzahl n_{d} = 1,5182 und eine Abbe-Zahl v_{d} = 58,9 auf. Das Material zwischen den Grenzflächen A3 und A4 weist eine Brechzahl von n_{d} = 1,6377 und eine Abbe-Zahl von v_{d} = 42,4 auf. Das Material zwischen den Grenzflächen A4 und A5 weist eine Brechzahl von n_{d} = 1,65 und eine Abbe-Zahl von v_{d} = 50,9 auf.

Die Stablinse vom Typ B weist die Grenzflächen B1 (asphärisch positiv gekrümmt), B2 (sphärisch negativ gekrümmt), B3 (sphärisch negativ gekrümmt), B4 (sphärisch positiv gekrümmt) und B5 (sphärisch negativ gekrümmt) auf. Das Material zwischen den Grenzflächen B1 und B2 hat eine Brechzahl n_{d} = 1,75 und eine Abbe-Zahl von v_{d} = 45,4. Das Material zwischen Grenzflächen B2 und B3 weist eine Brechzahl von n_{d} = 1,523 und eine Abbe-Zahl von v_{d} = 59,5 auf. Das Material zwischen den Grenzflächen B3 und B4 weist eine Brechzahl n_{d} = 1,6377 und eine Abbe-Zahl v_{d} = 42,4 auf. Das Material zwischen den Grenzflächen B4 und B5 weist eine Brechzahl von n_{d} = 1,65 und eine Abbe-Zahl von v_{d} = 50,9 auf.

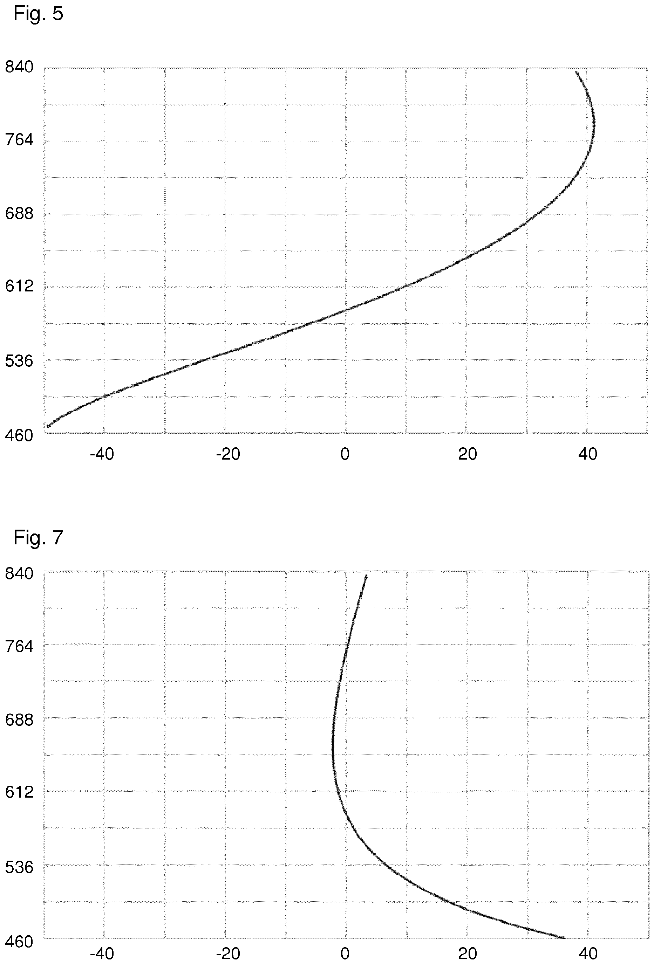

Ein derart ausgebildetes Umkehrsystem 6 weist ein Farblängsfehlerverhalten auf, wie in Fig. 5 gezeigt ist. In Fig. 5 ist in gleicher Weise wie in Fig. 3 die Fokuslage entlang der x-Achse in µm und die Wellenlänge entlang der y-Achse in nm aufgetragen. Dieser Darstellung in Fig. 5 kann entnommen werden, dass der Farblängsfehler für eine erste vorbestimmte Wellenlänge von 540 nm aus dem sichtbaren Wellenlängenbereich bezogen auf eine zweite vorbestimmte Wellenlänge von 840 nm aus dem nahen Infrarotbereich ca. 59,05 µm beträgt. Für die Wellenlängen 460 nm und 840 nm beträgt der Farblängsfehler ca. 88,08 µm. Das Farblängsfehlerverhalten kann als positiver Farblängsfehler beschrieben werden.

Das Objektiv 4 weist den in Fig. 6 gezeigten Aufbau mit einem Planglas 22, einem Prisma 23 und Linsen 24-30 auf. Das Prisma 23 ist in der Darstellung in Fig. 6 als gestreckter Glasblock dargestellt, der bei der Ausbildung des Optiksystems für ein Geradeausblick-Endoskop, wie in Fig. 1 gezeigt ist, als gezeigter Glasblock realisiert wird. Bei der Ausführung des Optiksystems 1 für eine geneigte Blickrichtung, wie in Fig. 2 gezeigt ist, ist das Prisma 23 dann tatsächlich als Prisma ausgebildet. Die gewählte Darstellung in Fig. 6 dient lediglich zur Vereinfachung der Darstellung, da es für die Beschreibung des Farblängsfehlers auf die Glaswege sowie die gekrümmten Grenzflächen ankommt.

Die Krümmungseigenschaften und verwendeten Materialien sind in der nachfolgenden Tabelle für die Flächen F1-F16 des Objektivs 4 angegeben, wobei die Angabe der Brechzahl n_{d} und der Abbe-Zahl v_{d} für eine Fläche bedeutet, dass dieses Material zwischen der Fläche, in dessen Zeile die Angaben sind, und der darauf folgenden nächsten Fläche vorliegt.

| Fläche | Krümmung | Brechzahl n_{d} | Abbe-Zahl v_{d} |
|---|---|---|---|
| F1 | plan | 1,768 | 72,2 |
| F2 | plan | | |
| F3 | sphärisch positiv | 1,892 | 37,1 |
| F4 | sphärisch positiv | | |
| F5 | plan | 1,835 | 42,7 |
| F6 | plan | 1,8502 | 32,2 |
| F7 | sphärisch negativ | | |
| F8 | sphärisch positiv | 1,529 | 77 |
| F9 | sphärisch negativ | | |
| F10 | sphärisch negativ | 1,855 | 24,8 |
| F11 | sphärisch negativ | | |
| F12 | sphärisch positiv | 1,603 | 65,4 |
| F13 | sphärisch negativ | 1,6377 | 42,4 |
| F14 | sphärisch positiv | | |
| F15 | sphärisch positiv | 1,529 | 77 |
| F16 | sphärisch negativ | | |

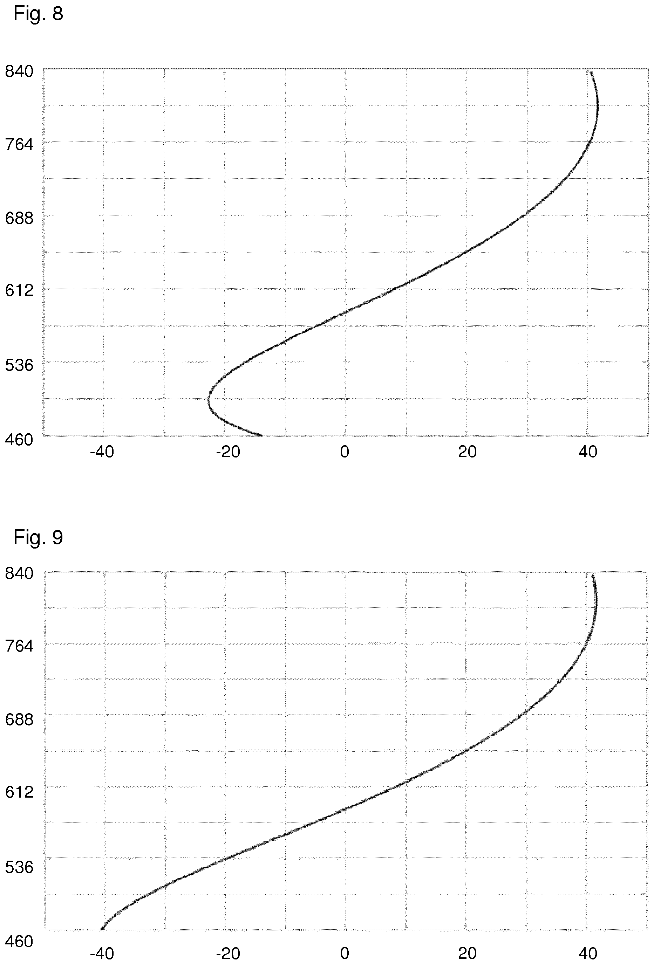

Der durch das Objektiv 4 bedingte Farblängsfehler ist in Fig. 7 in gleicher Weise wie in Fig. 5 dargestellt. Das Objektiv 4 wurde so ausgelegt, dass ein im Wesentlichen negativer Farblängsfehler vorliegt. So ist der Farblängsfehler für die Wellenlänge 540 nm und 840 nm -2,3 µm. Für die Wellenlängen 460 nm und 840 nm beträgt er -32,7 µm. Der durch das Objektiv 4 und das Umkehrsystem 8 in der Summe bei der Abbildung von der distalen Zwischenbildebene 7 in die proximale Zwischenbildebene 9 eingepragte Farblängsfehler ist in Fig. 8 gezeigt. Aufgrund der entgegengesetzten Farblängsfehler von Objektiv 4 und Umkehrsystem 8 ist der resultierende Farblängsfehler geringer als der des Umkehrsystems 8 alleine. So beträgt der Farblängsfehler für die Wellenlänge 540 und 840 nm ca. 55,61 µm und für die Wellenlängen 460 und 840 nm ca. 54,05 µm. Aufgrund dieses äußerst geringen Farblängsfehlers sind scharfe Aufnahmen mit Licht aus dem sichtbaren Wellenlängenspektrum und mit Licht aus dem Infrarotspektrum problemlos möglich.

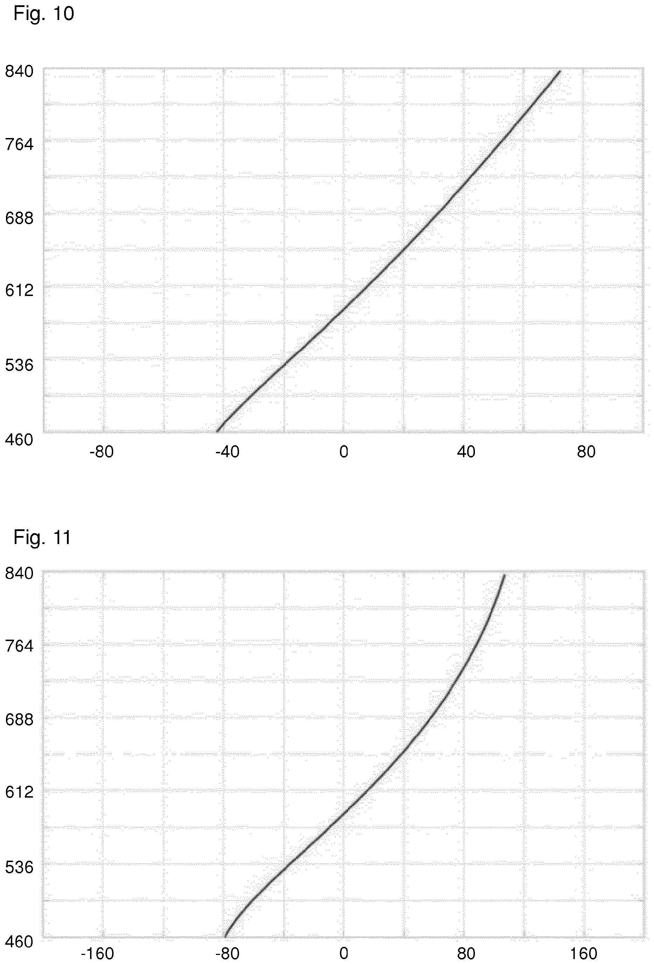

In Fig. 9-11 sind der Farblängsfehler eines Umkehrsystems (Fig. 9), der Farblängsfehler eines Objektivs (Fig. 10) und der resultierende Farblängsfehler von Objektiv und Umkehrsystem (Fig. 11) eines bekannten Vergleichsendoskopes (das auch ein Optiksystem aus Objektiv und Umkehrsystem in einem starren Endoskopschaft aufweist) in gleicher Weise wie in Fig. 5-8 gezeigt. Das Vergleichsendoskop wurde hinsichtlich eines möglichst kleinen Farblängsfehlers des Umkehrsystems ausgelegt. Dieser ist auch geringfügig geringer als der des Umkehrsystems 8 des erfindungsgemäßen Optiksystems. So beträgt der Farblängsfehler des Umkehrsystems des Vergleichsendoskopes für 540 und 840 nm ca. 59 µm und für 460 und 840 nm ca. 82,1 µm. Dies wird durch die Verwendung vieler unterschiedlicher Gläser erreicht, da das Umkehrsystem des Vergleichsendoskops drei Umkehrstufen à zwei Stablinsen mit jeweils 4-5 Elementen aufweist, so dass insgesamt 27 Elemente vorgesehen sind. Ferner sind die Brechzahlsprünge zwischen den verschiedenen Materialien größer als 0,5.

Das Objektiv des Vergleichsendoskops weist den positiven Farblängsfehler gemäß Fig. 10 auf. So beträgt der Farblängsfehler für 540 und 840 nm ca. 88,7 µm und für 460 und 840 nm ca. 115,5 µm. Dies ist zwar ein kleiner Farblängsfehler, jedoch ist er positiv, so dass der resultierende Farblängsfehler, wie in Fig. 11 gezeigt ist, für 540-840 nm ca. 140 µm und für 460-840 nm ca. 186,7 µm beträgt. Damit ist der resultierende Farblängsfehler deutlich größer als bei dem erfindungsgemäßen Optiksystem.

Des Weiteren führt die Verwendung von mehr optischen Elementen im Umkehrsystem und von Materialien mit Brechzahlsprüngen von größer als 0,5 im Umkehrsystem dazu, dass größere Fresnel-Verluste im Vergleich zu dem erfindungsgemäßen Umkehrsystem 8, bei dem die Brechzahlsprünge nicht größer als 0,3 sind, auftreten. Wegen der kleineren Brechzahlsprünge und der geringeren Anzahl von Linsen beim erfindungsgemäßen Umkehrsystem 8 ist die Transmission des erfindungsgemäßen Optiksystems 1 deutlich höher. So beträgt die Transmission bei dem erfindungsgemäßen Optiksystem 1 für die Wellenlängen 460 nm, 540 nm, 588 nm, 640 nm, 656 nm und 840 nm 0,69, 0,77, 0,78, 0,77, 0,76 sowie 0,78. Das gibt eine Gesamttransmission von 0,75. Bei dem Optiksystem des bekannten Endoskopes beträgt die Transmission für die gleichen Wellenlängen hingegen lediglich 0,60, 0,71, 0,72, 0,72, 0,72 und 0,74. Die Gesamttransmission beträgt dann 0,68. Somit liegt bei dem erfindungsgemäßen Optiksystem 1 eine um 16 % größere Helligkeit für blau (640 nm), eine um 9 % größere Helligkeit für grün (540 nm) und eine um 5 % größere Helligkeit für nahes Infrarot 840 nm vor. Aufgrund der höheren Blautransmission ist darüber hinaus das Bild weißer, was einen besseren Bildeindruck mit sich bringt.

## Patentansprüche

1. Optiksystem für ein Endoskop, mit
einem Objektiv (4) zum Abbilden eines Objektes (6) als distales Zwischenbild in eine distale Zwischenbildebene (7) und
einem dem Objektiv (4) nachgeordneten und mindestens eine Umkehrstufe (17, 18, 19) aufweisenden Umkehrsystem (8) zum Abbilden des distalen Zwischenbildes als proximales Zwischenbild in eine proximale Zwischenbildebene (9),
wobei das Umkehrsystem (8) dem proximalen Zwischenbild einen ersten Farblängsfehler bezogen auf eine vorbestimmte Wellenlänge aus dem sichtbaren Spektrum und eine vorbestimmte Wellenlänge aus dem nahen Infrarotbereich einprägt,
wobei das Objektiv (4) dem distalen Zwischenbild einen zweiten Farblängsfehler bezogen auf die vorbestimmte Wellenlänge aus dem sichtbaren Spektrum und die vorbestimmte Wellenlänge aus dem nahen Infrarotbereich einprägt,
und wobei der zweite Farblängsfehler relativ zum ersten Farblängsfehler ein entgegengesetztes Vorzeichen aufweist, um den vom Umkehrsystem (8) bedingten Farblängsfehler im proximalen Zwischenbild zu verringern,
wobei die vorbestimmte Wellenlänge aus dem sichtbaren Spektrum 540 nm und die vorbestimmte Wellenlänge aus dem nahen Infrarotbereich 840 nm beträgt,
**dadurch gekennzeichnet, dass**
der erste Farblängsfehler im Bereich von 35 bis 65 µm und der zweite Farblängsfehler im Bereich von -4 bis -0,5 µm liegt.

2. Optiksystem nach Anspruch 1, bei dem alle gekrümmten Materialgrenzflächen des Objektivs (4) sphärisch gekrümmte sind.

3. Optiksystem nach Anspruch 1 oder 2, bei dem das Umkehrsystem (18) mehrere hintereinander angeordnete Umkehrstufen (17-19) aufweist.

4. Optiksystem nach einem der obigen Ansprüche, bei dem die mindestens eine Umkehrstufe (17-19) zwei direkt miteinander verbundene Linsen aufweist, deren Materialien so gewählt sind, dass sich die Brechzahlen der Materialien um nicht mehr 0,3 unterscheiden.

5. Optiksystem nach Anspruch 4, bei dem jeder Brechzahlsprung aller direkt miteinander verbundenen Linsen von mindestens einer Umkehrstufe (17-19) kleiner oder gleich 0,3 ist.

6. Optiksystem nach einem der obigen Ansprüche, bei dem mindestens eine Umkehrstufe eine Linse mit einer asphärischen Grenzfläche aufweist.

7. Optiksystem nach einem der obigen Ansprüche, bei dem die vorbestimmte Wellenlänge aus dem sichtbaren Spektrum im Bereich von 400-700 nm und die vorbestimmte Wellenlänge aus dem nahen Infrarotbereich im Bereich von 710-900 nm liegt.

8. Optiksystem nach einem der obigen Ansprüche, bei dem
das Umkehrsystem einen dritten Farblängsfehler für die Wellenlänge von 540 nm zur Wellenlänge von 460 nm und einen vierten Farblängsfehler für die Wellenlänge von 540 nm zur Wellenlänge von 640 nm aufweist,
das Objektiv einen fünften Farblängsfehler für die Wellenlänge von 540 nm zur Wellenlänge von 460 nm und einen sechsten Farblängsfehler für die Wellenlänge von 540 nm zur Wellenlänge von 640 nm aufweist,
wobei das Verhältnis des dritten zum vierten Farblängsfehler im Bereich von -1,1 bis -0,8 und das Verhältnis des fünften zum sechsten Farblängsfehler im Bereich von -4,5 bis -3 liegt.

9. Optiksystem nach einem der obigen Ansprüche, bei dem
das Objektiv einen siebten Farblängsfehler für die Wellenlänge von 460 nm zur Wellenlänge von 840 nm und einen Bildwinkel alpha im Objektraum aufweist,
wobei das Produkt von sin(alpha/2) mit dem siebten Farblängsfehler im Bereich von 18 bis 26 µm liegt.

10. Optiksystem nach einem der obigen Ansprüche, bei dem
die vorbestimmte Wellenlänge aus dem sichtbaren Spektrum 540 nm und die vorbestimmte Wellenlänge aus dem nahen Infrarotbereich 840 nm beträgt und das Umkehrsystem n optische Elemente aufweist,
wobei das Produkt aus der Anzahl n der optischen Elemente mit dem ersten Farblängsfehler im Bereich von 1500 bis 2000 µm liegt.

11. Optiksystem nach einem der obigen Ansprüche, bei dem das Objektiv dem distalen Zwischenbild einen solchen Farblängsfehler einprägt, dass die Fokuslage für eine Wellenlänge von 840 nm zwischen den Fokuslagen für 540 nm und 640 nm liegt.

12. Endoskop mit einem Optiksystem nach einem der obigen Ansprüche.

## Claims

1. Optical system for an endoscope, having
an objective (4) for imaging an object (6) as a distal intermediate image in a distal intermediate image plane (7) and
a reversal system (8) arranged after the objective (4) and comprising at least one reversal stage (17, 18, 19) for projecting the distal intermediate image as a proximal intermediate image into a proximal intermediate image plane (9),
wherein the reversal system (8) imprints on the proximal intermediate image a first longitudinal chromatic aberration referred to a predetermined wavelength from the visible spectrum and a predetermined wavelength from the near infrared range,
wherein the objective (4) imprints on the distal intermediate image a second longitudinal chromatic aberration referred to the predetermined wavelength from the visible spectrum and the predetermined wavelength from the near infrared range,
and wherein the second longitudinal chromatic aberration has the opposite sign relative to the first longitudinal chromatic aberration in order to reduce the longitudinal chromatic aberration caused by the reversal system (8) in the proximal intermediate image,
wherein the predetermined wavelength from the visible spectrum is 540 nm and the predetermined wavelength from the near infrared range is 840 nm,
**characterized in that**,
the first longitudinal chromatic aberration lies in the range from 35 to 65 µm and the second longitudinal chromatic aberration lies in the range from -4 to -0.5 µm.

2. Optical system according to claim 1, in which all curved material boundary surfaces of the objective (4) are spherically curved.

3. Optical system according to claim 1 or 2, in which the reversal system (18) comprises several reversal stages (17-19) arranged one behind the other.

4. Optical system according to one of the above claims, in which the at least one reversal stage (17-19) comprises two lenses directly joined to each other, the materials of which are chosen such that the refractive indices of the materials differ by not more than 0.3.

5. Optical system according to claim 4, in which each refractive index step of all lenses which are directly joined to each other from at least one reversal stage (17-19) is smaller than or equal to 0.3.

6. Optical system according to one of the above claims, in which at least one reversal stage comprises a lens with an aspherical boundary surface.

7. Optical system according to one of the above claims, in which the predetermined wavelength from the visible spectrum is in the range of 400-700 nm and the predetermined wavelength from the near infrared range is in the range of 710-900 nm.

8. Optical system according to one of the above claims, wherein
the reversal system comprises a third longitudinal chromatic aberration for the wavelength of 540 nm relative to the wavelength of 460 nm and a fourth longitudinal chromatic aberration for the wavelength of 540 nm relative to the wavelength of 640 nm,
wherein the objective comprises a fifth longitudinal chromatic aberration for the wavelength of 540 nm relative to the wavelength of 460 nm and a sixth longitudinal chromatic aberration for the wavelength of 540 nm relative to the wavelength of 640 nm,
wherein the ratio of the third longitudinal chromatic aberration to the fourth longitudinal chromatic aberration lies in the range from -1.1 to -0.8 and the ratio of the fifth longitudinal chromatic aberration to the sixth longitudinal chromatic aberration lies in the range from -4.5 to -3.

9. Optical system according to one of the above claims, wherein
the objective comprises a seventh longitudinal chromatic aberration for the wavelength of 460 nm relative to the wavelength of 840 nm and an image angle alpha in the object space, wherein the product of sin(alpha/2) and the seventh longitudinal chromatic aberration lies in the range from 18 to 26 µm.

10. Optical system according to one of the above claims, wherein
the predetermined wavelength from the visible spectrum is 540 nm and the predetermined wavelength from the near infrared range is 840 nm and the reversal system comprises n optical elements,
wherein the product of the number n of optical elements and the first longitudinal chromatic aberration lies in the range from 1500 to 2000 µm.

11. Optical system according to one of the above claims, wherein the objective imprints on the distal intermediate image such a longitudinal chromatic aberration that the focus position of a wavelength of 840 nm lies between the focus positions for 540 nm and 640 nm.

12. Endoscope with an optical system according to one of the above claims.

## Revendications

1. Système optique destiné à un endoscope, ledit système optique comprenant
un objectif (4) destiné à la reproduction d'un objet (6) comme image intermédiaire distale dans un plan d'image intermédiaire distal (7) et
un système d'inversion (8) qui est disposé en aval de l'objectif (4), qui comporte au moins un étage d'inversion (17, 18, 19) et qui est destiné à reproduire l'image intermédiaire distale comme image intermédiaire proximale dans un plan d'image intermédiaire proximal (9),
le système d'inversion (8) imprimant à l'image intermédiaire proximale une première aberration chromatique longitudinale liée à une longueur d'onde prédéterminée du spectre visible et à une longueur d'onde prédéterminée du domaine des infrarouges proches,
l'objectif (4) imprimant à l'image intermédiaire distale une deuxième aberration chromatique longitudinale liée à la longueur d'onde prédéterminée du spectre visible et à la longueur d'onde prédéterminée du domaine des infrarouges proches,
et la deuxième aberration chromatique axiale ayant un signe opposé à celui de la première aberration chromatique axiale afin de réduire dans l'image intermédiaire proximale l'aberration chromatique longitudinale due au système d'inversion (8),
la longueur d'onde prédéterminée du spectre visible étant de 540 nm et la longueur d'onde prédéterminée du domaine des infrarouges proches étant de 840 nm, **caractérisé en ce que**
la première aberration chromatique axiale est dans la gamme allant de 35 à 65 µm et la deuxième aberration chromatique axiale est dans la gamme allant de -4 à - 0,5 µm.

2. Système optique selon la revendication 1, dans lequel toutes les surfaces limites de matériau incurvées de l'objectif (4) présentent une courbure sphérique.

3. Système optique selon la revendication 1 ou 2, dans lequel le système d'inversion (18) comporte une pluralité d'étages d'inversion (17-19) disposés les uns derrière les autres.

4. Système optique selon l'une des revendications précédentes, dans lequel l'au moins un étage d'inversion (17-19) comporte deux lentilles qui sont reliées directement l'une à l'autre et dont les matériaux sont choisis de telle sorte que les indices de réfraction des matériaux ne diffèrent pas de plus de 0,3.

5. Système optique selon la revendication 4, dans lequel chaque saut d'indice de réfraction de toutes les lentilles reliées directement les unes aux autres d'au moins un étage d'inversion (17-19) est inférieur ou égal à 0,3.

6. Système optique selon l'une des revendications précédentes, dans lequel au moins un étage d'inversion comporte une lentille pourvue d'une surface limite asphérique.

7. Système optique selon l'une des revendications précédentes, dans lequel la longueur d'onde prédéterminée du spectre visible est dans la gamme allant de 400 à 700 nm et la longueur d'onde prédéterminée du domaine des infrarouges proches est dans la gamme allant de 710 à 900 nm.

8. Système optique selon l'une des revendications précédentes, dans lequel
le système d'inversion présente une troisième aberration chromatique axiale pour la longueur d'onde de 540 nm par rapport à la longueur d'onde de 460 nm et une quatrième aberration chromatique axiale pour la longueur d'onde de 540 nm par rapport à la longueur d'onde de 640 nm,
l'objectif présentant une cinquième aberration chromatique axiale pour la longueur d'onde de 540 nm par rapport à la longueur d'onde de 460 nm et une sixième aberration chromatique axiale pour la longueur d'onde de 540 nm par rapport à la longueur d'onde de 640 nm,
le rapport de la troisième à la quatrième aberration chromatique axiale étant dans la gamme allant de -1,1 à -0,8 et le rapport de la cinquième à la sixième aberration chromatique axiale étant dans la gamme allant de -4,5 à -3.

9. Système optique selon l'une des revendications précédentes, dans lequel
l'objectif présente une septième aberration chromatique longitudinale pour la longueur d'onde de 460 nm par rapport à la longueur d'onde de 840 nm et un angle de vue alpha dans l'espace objet,
le produit de sin(alpha/2) et de la septième aberration chromatique axiale étant dans la gamme allant de 18 à 26 µm.

10. Système optique selon l'une des revendications précédentes, dans lequel
la longueur d'onde prédéterminée du spectre visible est de 540nm et la longueur d'onde prédéterminée du domaine des infrarouges proches est de 840 nm et le système d'inversion comporte n éléments optiques,
le produit du nombre n d'éléments optiques et de la première aberration chromatique axiale étant dans la gamme allant de 1500 à 2000 µm.

11. Système optique selon l'une des revendications précédentes, dans lequel l'objectif imprime à l'image intermédiaire distale une aberration chromatique longitudinale telle que la position focale pour une longueur d'onde de 840 nm est située entre les positions focales pour 540 nm et 640 nm.

12. Endoscope comprenant un système optique selon l'une des revendications précédentes.
